Europäisches Patentamt

European Patent Office

Office européen des brevets

(11) Publication number: **0 300 512**
**A2**

# EUROPEAN PATENT APPLICATION

(21) Application number: 88112710.4

(22) Date of filing: 23.09.85

(51) Int. Cl.⁴: **A61F 2/24**

(30) Priority: 24.09.84 US 653960

(43) Date of publication of application:
**25.01.89 Bulletin 89/04**

(60) Publication number of the earlier application in accordance with Art.76 EPC: **0 176 337**

(84) Designated Contracting States:
**AT BE CH DE FR GB IT LI NL SE**

(71) Applicant: **Carbomedics Inc.**
**1300 East Anderson Lane**
**Austin Texas 78752(US)**

(72) Inventor: **Bokros, Jack Chester**
**2204 Manana Street**
**Austin Texas 78732(US)**

(74) Representative: **Bizley, Richard Edward et al**
**BOULT, WADE & TENNANT 27 Furnival Street**
**London EC4A 1PQ(GB)**

(54) **Heart valve prothesis.**

(57) A heart valve prostheses (121) comprising a generally annular body (123) which has an interior surface (127) that defines a central passageway for blood flow therethrough, occluder means (125) supported on said body for alternately blocking and then allowing the flow of blood through said passageway in a predetermined direction, said occluder means (125) being formed with means at opposite locations in the periphery thereof that cooperate with complementary means interior of said annular body, and a pair of opposed seat means (161,163) provided on the interior surface (127) of an annular body (123), each of said seat means (161,163) having a surface which is generally that of a portion of a frustum of a cone so that when the periphery of the occluder (125) engages said pair of seat means (161,163) in the closed position a good seal is achieved therealong.

Fig.1.

# HEART VALVE PROSTHESIS

This invention relates to heart valve prostheses for replacement of defective natural valves and more particularly to heart valve prostheses which employ one or more occluders in the form of flat platelike members, although certain features of the invention are applicable to valves having curved occluders.

Various types of heart valve prostheses have been developed which operate hemodynamically as a result of the pumping action of the heart and which are in essence functioning as check valves. Early heart valves employed a ball-and-cage arrangement whereas later versions of heart prostheses have employed one or more occluders generally in the form of a plate or disc which might be flat or of a curved shape. Bokros Patent No. 3546711 shows a heart valve having a circular occluder which is pivoted from a hinge pin that coacts with a pair of upstanding fins located on the downstream surface of the occluder. Bokros Patent No. 4178639 shows a bi-leaflet heart valve having a pair of platelike members each of which has ears extending from its opposite lateral edges that pivot in spheroidal guides located in the orifice ring. U.S. Patent No. 3445863 shows a heart valve arrangement having one or more occluders in the form of flat plates having cut-outs at their edges which are proportioned to interfit with complementary cut-outs in a base ring formed of generally similar material. U.S. Patent No. 4225980 shows a metallic heart valve which includes an oval-shaped occluder having cut-outs in oppose lateral edges which coact with parabolic pivots or pegs that extend radially inward from the interior surface of the orifice ring. US Patent No. 4078268 shows a bi-leaflet valve having a pair of semicircular occluders which have ears extending upward from the downstream surface thereof that co-act with fulcrums to effect a pivoting action. U.S. Patent No. 4159543 shows a variety of bi-leaflet valves, one version of which has grooves cut into the edges of the semicircular occluders at an oblique angle to the surface thereof, which grooves receive generally conical pivot pins that extend radially inward from the interior surface of the orifice ring. U.S. Patent No. 4373216 illustrates heart valves having one or more occluders that have aligned notch means in opposite edges of the periphery thereof which co-act with elongated aligned protuberances extending inward from the surface of the orifice ring, these protuberances having arcuate opposite lateral surfaces, and which are proportioned to be received within the notch means, and provide tracks for guiding pivotal and translational movement of the occluders.

As is apparent from the foregoing, a wide variety of different heart valves have been designed, and work continues on new heart valve designs in order to still farther improve the functioning of these prostheses which are being used in greater quantity each year as surgical techniques improve throughout the world.

The invention provides a heart valve prostheses comprising a generally annular body which has an interior surface that defines a central passageway for blood flow therethrough, occluder means supported on said body for alternately blocking and then allowing the flow of blood through said passageway in a predetermined direction, said occluder means being formed with means at opposite locations in the periphery thereof that cooperate with complementary means interior of said annular body, and a pair of opposed seat means provided on the interior surface of said annular body, each of said seat means having a surface which is generally that of a portion of a frustum of a cone so that when the periphery of the occluder engages said pair of seat means in the closed position a good seal is achieved therealong.

In copending application No. 85306754.4, from which this application is divided, there is described and claimed a heart valve prosthesis comprising a generally annular body which has an interior surface that defines a central passageway for blood flow therethrough, occluder means supported on said body for alternately blocking and then allowing the flow of blood through said passageway in a predetermined direction, said occluder means being formed with aligned notch means at opposite locations in the periphery thereof, a pair of aligned projections projecting generally radially inward from said interior surface of said body, which projections have arcuate opposite lateral surfaces and are proportioned to be received within said notch means, and stop means projecting inward from said interior surface at locations generally flanking each of said projections characterised in that said projections are pivot posts for said occluder means and that said stop means extend radially farther inward than said pivots, said stop means together presenting a pair of curved surfaces in regions adjacent to said pivot posts, each of which curved surfaces lies in a region located intermediate of said opposite arcuate lateral surfaces of said pivot posts and radially inward thereof so as to provide a pair of oppositely disposed fulcrums that alternately assist in defining the opening and closing movement of said occluder means.

## BRIEF DESCRIPTION OF THE DRAWINGS

Figure 1 is a perspective view of a heart valve embodying various features of the invention, shown without the occluder means.

Figure 2 is a fragmentary plan view of the annular body shown in Figure 1.

Figure 3 is a perspective view of the heart valve showing the annular body of Figure 1 with the occluder installed and in the open position.

Figure 4 is a side view of the occluder with a portion broken away to show its internal construction.

Figure 5 is a fragmentary front view of the occluder.

Figure 6 is a sectional view of the heart valve shown in Figure 3 depicting in full lines the occluder in an intermediate position as it is moving to the closed position and with the occluder shown twice in broken lines in the position which it assumes in the open position and in the closed position.

Figure 7 is an enlarged fragmentary view of the interior surface of the annular ring emphasising the pivot support area.

## DETAILED DESCRIPTION OF THE PREFERRED EMBODIMENT

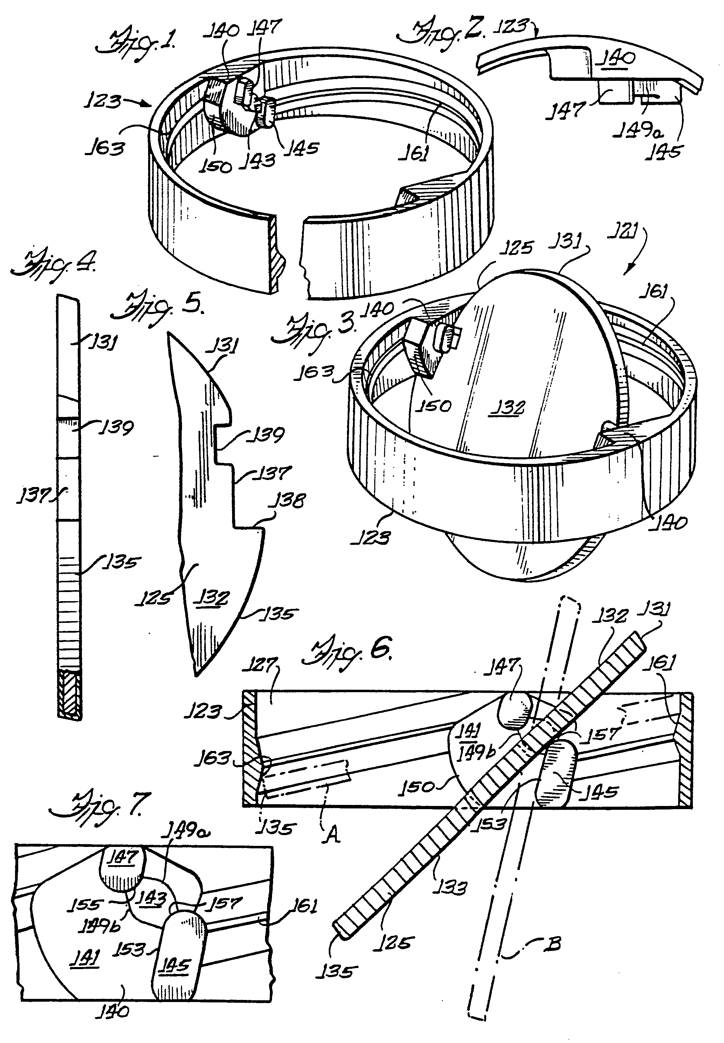

Depicted in Figures 1 to 7 of the drawings is a preferred embodiment of a heart valve 121 which includes an annular body 123 designed to operate with a singular flat occluder or disc 125.

Illustrated in Figures 1 and 3 is a heart valve 121 which consists of an annular valve body or housing 123 which supports a pivoting occluder 125 that opens and closes to control the blood flow through a central passageway or orifice which is defined by the interior surface 127 of the valve body. Inasmuch as the annular body 123 defines the central passageway or orifice, it is sometimes referred to as an orifice-ring. The normal flow of blood through the heart valve 121 is downward in the orientation in which the valve is shown in Figures 1 and 3. It should, of course, be understood that the valve 121 can operate in any orientation and is not significantly affected by gravity. Thus, the terms such as upward and downward, as used hereinafter, are merely employed to facilitate explanation and understanding and are not meant to place any limitations upon the operation of the heart valves being described.

The illustrated occluder 125 is flat and has a uniform thickness throughout. The occluder 125 has a straight-edge portion 131, which is best seen in Figure 4 and which is oriented at an angle to the downstream surface 133 of the occluder so that the straight-edge portion 131 contacts with the inwardly extending seats 161, 163. Generally, the angle will be between about 110° and about 130°, being of course dependent upon the orientation of the occluder to the horizontal or transverse plane in the closed position.

The annular body 123 has an interior surface 127 which defines the passage or orifice through which the bloodstream will flow and is illustrated with a smooth exterior cylindrical surface 129, which surface is that of the lateral surface of a right circular cylinder. It should be understood that suitable means, such as a peripheral groove or a pair of flats, would usually be provided for attaching a suturing ring to the annular body to facilitate sewing or suturing of the heart valve 21 to the heart tissue. However, inasmuch as the suturing ring and its means of attachment to the heart valve 121 form no part of the present invention, it is simply omitted so as to facilitate the illustration of the specific components of the heart valve with which the invention is concerned. US Patent No. 4233690, issued November 18, 1980, illustrates one method of attaching a suturing ring to an annular heart valve body.

The interior surface 127 has inwardly extending seats 161, 163 against which the arcuate edges of the disc occluder 125 can positively seat.

The valve body 123 and the occluder 125 may be made of any suitable material that is biocompatible and nonthrombogenic and that will take the wear to which it will be subjected during countless opening and closing movements of the occluder. Preferably, the occluder is made of isotropic graphite, such as that sold under the tradename POCO and that preferably contains between about 5 and about 20 weight percent tungsten to render it radio-opaque and allow x-ray visualisation, which graphite has been suitably coated with pyrolytic carbon, such as that sold under the trademark PYROLITE. Such pyrocarbon gives excellent blood and tissue compatibility and wear-resistance.

The orifice ring 123 can be made in the same fashion using a pyrocarbon-coated substrate, or it can be, and preferably is, made from solid PYROLITE pyrocarbon. A preferred process for forming an orifice ring from all pyrocarbon by coating a mandrel, which is subsequently removed, is disclosed in European Patent Application, Publication No. 0055406 Al.

Support means for the occluder 125 includes a pair of pivot posts 143, which are sized to fit within notches 139 and which pivot posts are flanked by stop means 145, 147. As can be seen from Figures 1 and 7, the flanking stop means 145, 147 project radially farther inward than do the pivot posts 143.

Preferably, the pivot posts 143 and the flanking stop means are formed integrally with one another, and most preferably this overall support structure is formed as an integral part of the annular body 123, as by machining a pre-shaped body of pyrolytic carbon to final dimensions. As best seen in Figure 7, the pivot posts 143 each have a pair of opposite arcuate surfaces, a generally upper surface 149a and a generally lower surface 149b, which are referred to as the lateral surfaces of the posts.

Guidance of the occluder is obtained by providing a pair of protrusions or lands 140 which extend from the inner surface of the annular body 123 and present a pair of oppositely disposed flat Surfaces 141. The protrusions 140 lie substantially to the right of a diameter of the passageway which is parallel to the pivot axis as viewed in Figure 6.

Projecting radially inwardly from each of these flat surfaces 141 is a pivot post 143 and a pair of stops 145, 147. The pivot posts 143 have arcuate, generally upper and lower surfaces 149a,b which are similarly formed with the focal point of the radius of curvature lying beyond the centre point of the pivot post and, if desired, past the opposite surface of the post, for the purpose described hereinafter.

The occluder 125 generally has the form of a flat disc having an upstream surface 132 and a downstream surface 133. The periphery of the disc includes a minor arcuate section 131, a major arcuate section 135 and a pair of oppositely disposed, straight, parallel intermediate sections 137. Notches 139 of generally rectangular shape are located in the regions of the straight edge portions 137. The shape of the major arcuate edge 135 is generally that of a section of an ellipse, and the shape of the minor arcuate edge section 131 is also generally that of a section of an ellipse with a slightly shorter minor axis. As best seen perhaps in Figure 5, the intermediate edge portions are each inwardly offset from the outermost lateral extension of the major edge surface by short perpendicular transitional surfaces 138 that are collinear. As best seen in Figure 4, the circular disc is also preferably made of isotropic graphite which has been coated with PYROLITE pyrolytic carbon.

The annular body 121 is suitably distended so as to allow the occluder 125 to be snapped into place with the notches 139 fitting about the pivot posts 143. The length of the pivot post 143 is just less than the depth of the notches 139 so that the flat edge surfaces 137 of the occluder will bear against the flat surface 141 of the protrusions and provide a bearing surface during pivoting between open and closed positions. In addition, each protrusion 140 is formed with a curved under surface 150 against which the transitional surface 138 wipes during pivoting action.

In the open position, as shown in Figure 3 and in broken lines in Figure 6, the occluder is oriented at between about 5° and 15° to the centreline of the passageway, e.g., at about 10°, lying generally against an inward-facing surface 153 of the stop 145 and with the upper surface of the notch 139 in contact with the upper surface 149a of the pivot post. There may also be contact between the upstream surface 132 of the occluder and the upper stop member 147.

As soon as the pumping stroke of the ventricle ceases for a valve in the aortic position, the back pressure flow lifts the occluder 125 so that the lower surface of the notch 139 contacts the arcuate surface 149b of the pivot post and the upstream surface 132 contacts a curved surface 155 formed on the stop 147 at a location intermediate of the upstream and downstream surfaces 149a,b of the pivot post 143, both of which surfaces 149b guide the pivoting motion. As pivoting movement continues to an intermediate position, the transitional edge surface 138 of the occluder essentially wipes along the curved under surface 150 of the protrusion. When the occluder 125 reaches the fully closed position (see broken line illustration "A" in Figure 6), its major and minor arcuate edges are in contact with the pair of generally semi-elliptical seats 161, 163 which are formed as a part of the annular body 123 projecting inward from its interior surface. Because each of these seats is shaped with a generally conical or oblique configuration, the occluder will be self-centering so long as there is clearance at the notches 139, and there will be line contact between each edge of the occluder and one of the seat surfaces, thus providing a more positive seal around the periphery of the occluder when the heart valve is in the closed position. This unique self-centering seating feature is applicable to other heart valve designs where a pair of opposed seats can be provided, each of which has a surface that is a portion of generally the surface of a frustum of a cone, one being conical upward and the other being conical downward.

When the ventricle again begins to contract to resume the next pumping stroke, the pressure against the upstream surface 132 causes pivoting to begin, and the flow of blood displaces the occluder 125 slightly downstream so that the upper edge of the notch is in contact with the upper arcuate surface 149a of the pivot post which, along with a curved upper surface 157 formed on the lower stop 145 (also intermediate of the upstream and downstream surfaces 149a and 149b), defines the path of pivoting movement from the closed to the open position. An intermediate position is illustrated in full lines in Figure 6, and the pivoting movement is terminated when the under surface 133 of the occluder comes into contact with the stop surface 153 in the fully open position, as shown in broken line outline "B" in Figure 6. In this position, there is excellent blood flow through the heart valve central passageway because, as best seen in Figure 3, the post and stop arrangement only extends minimally into the passageway region and because the additional vacant area adjacent the surface 150 of the protrusion further decreases the resistance to flow through the valve.

By forming the posts 143 and stop means 145, 147 integral with one another, the regions of joinder can be blended together arcuately so there are no sharp depressions or valleys, i.e. no regions of concave curvature having a radius of curvature less than about 0.2 mm, which would be locations ripe for the beginning of clotting; thus, there is a substantial advantage gained from being able to have such a smooth transition from surface-to-surface, particularly where the very ends of the arcuate surfaces are not necessary for guidance. Moreover, the desired smooth swinging of the occluder between the open and closed positions is farther facilitated by the radius of curvature which appears on the surfaces 149a and 149b of the pivot posts 143. In this respect, the focal point of the radius of curvature of each of these surfaces 149a, 149b should lie beyond the centre point of the pivot post and may even lie past the opposite side of the pivot post.

The streamlined configuration provided by the integral post and stop means, as best seen perhaps in Figure 2, provides a minimum of disruption to flow through the central passageway. Moreover, the integral construction allows one curved surface to be blended into the adjacent curved surface thus avoiding the creation of crevices and/or valleys that tend to permit stagnation and promote blood clotting.

Although the invention has been described with regard to a preferred embodiment, it should be understood that various changes and modifications as would be obvious to one having the ordinary skill in this art may be made without departing from the scope of the invention which is defined by the claim appended hereto. For example, with respect to many of the novel features of the invention, the occluder need not be flat but could be curved in cross-section as have been shown in a number of prior art heart valve designs.

This specification includes, by reference, all the subject matter of European Patent Application No. 85306754.5 and thus includes heart valve prostheses having the seat means as described herein in conjunction with more than one occluder, and in particular occluder structures as described and/or claimed therein.

## Claims

1. A heart valve prostheses (121) comprising a generally annular body (123) which has an interior surface (127) that defines a central passageway for blood flow therethrough, occluder means (125) supported on said body for alternately blocking and then allowing the flow of blood through said passageway in a predetermined direction, said occluder means (125) being formed with means at opposite locations in the periphery thereof that cooperate with complementary means interior of said annular body, and a pair of opposed seat means (161,163) provided on the interior surface (127) of an annular body (123), each of said seat means (161,163) having a surface which is generally that of a portion of a frustum of a cone so that when the periphery of the occluder (125) engages said pair of seat means (161,163) in the closed position a good seal is achieved therealong.

EP 0 300 512 A2

*Fig. 1.* *Fig. 2.* *Fig. 3.* *Fig. 4.* *Fig. 5.* *Fig. 6.* *Fig. 7.*